# EUROPEAN PATENT SPECIFICATION

(11) **EP 2 996 628 B1**
(45) Date of publication and mention of the grant of the patent: **09.03.2022**
(21) Application number: 14723451.2
(22) Date of filing: 13.05.2014
(51) Int. Cl.: A61F 2/00

(54) **SURGICAL IMPLANT COMPRISING A LAYER HAVING OPENINGS**
CHIRURGISCHES IMPLANTAT MIT EINER SCHICHT MIT ÖFFNUNGEN
IMPLANT CHIRURGICAL POURVU D'UNE COUCHE COMPORTANT DES OUVERTURES

(30) Priority: 14.05.2013 DE 102013208924
(43) Date of publication of application: 23.03.2016
(73) Proprietor: Johnson & Johnson Medical GmbH, 22851 Norderstedt (DE)
(72) Inventor: PRIEWE, Jörg, 24113 Kiel (DE)
(74) Representative: Carpmaels & Ransford LLP
(86) International application number: PCT/EP2014/059762
(87) International publication number: WO 2014/184190

(56) References cited:
- WO-A1-03/099160
- WO-A1-2010/086515
- WO-A2-2008/045635
- FR-A1- 2 914 179
- US-A- 6 106 558

## Description

The present invention relates to a surgical implant for repair of a tissue or muscle wall defect, such as a ventral hernia. The disclosure provides processes for manufacturing such an implant. Furthermore, the disclosure provides a method of implanting the surgical implant.

Many ventral hernia repair implants comprise multiple mesh layers forming pockets or peripheral overlapping edges. The purpose of the pockets or overlapping edges is to enable a fastening instrument to fasten the implant through the parietal facing layer while maintaining a solid visceral facing layer to cover the hernia defect. While convenient for the surgical procedure, the implant construction requires additional material for fastening the implant, which therefore results in more foreign bodies in the patient. Further, a dual layer approach can result in a buckled configuration as tissue ingrowth may take place quickly in the parietal facing layer followed by tissue contraction in this layer, while the same may not occur in the visceral facing layer. The implant therefore needs to be properly fastened to the bodily tissue via the parietal layer.

WO2009/049910 A1 teaches a soft tissue repair implant which includes a body having a base section and at least one appendage extending outwardly from the base section. Again, such an implant contains additional material for fastening the implant.

WO99/45860 A1 describes implants that have micrometer sized depressions in their surfaces. The depressions are produced by means of lithographic processes or etching. The depressions prevent the adhesion to visceral tissue.

WO03/099160 A1 describes medical implants that are provided with bulges (protrusions or knobs) . It is mentioned that the knobbed film of the implant may be provided with holes so that bodily fluids can pass through the barrier created by the knobbed film. The knobs are orientated to the intestine.

US2012/0016388 A1 teaches an implantable plate comprising a textile support and having protuberances on its parietal face. When the plate is implanted, the protuberances provide friction between the plate and parietal tissue, thereby preventing displacement of said plate relative to said tissue.

The height of the protuberances according to US2012/0016388 A1 can be greater than the thickness of the support, but if the height exceeds three times the thickness of the support there is said to be a risk of aggressiveness of the fibres or filaments constituting said protuberances. Furthermore, the support can have an anti-adherent coating on the visceral face. US2012/0016388 A1 further teaches that the protuberances may have perforations which allow for drainage of body fluids and for tissue colonization of the plate. Suitable dimensions of the perforations are a diameter of 1 to 2 mm.

US2002/0183845 A1 describes a composite implant that creates an interface between a hard material, such as bone, and a soft and weak material, such as a gel or hydrogel, in which a non-planar layer with several holes (which is preferably covered by a hydrogel) is attached to another layer which is planar. The implant is used to replace damaged cartilage in a joint. The anchoring layer of the implant is secured to the hard surface using pins and/or cement.

US6,106,558 B1 describes columns on a neurone compression device. The columns or fins on the device are preferably produced by casting, and they reduce scar tissue formation subsequent to a nerve decompression procedure.

EP0898944 A2 describes a mesh that has a collar and which is attached by this to a second mesh. Again, this introduces a relatively large amount of material into the patient's body.

WO03/002029 A1 describes an implantable mesh that has at least one groove as a strengthening element. The grooves run across large parts of the mesh or run around it and expand the mesh from its collapsed shape.

WO2006/092159 A9 teaches a surgical implant with an areal base structure and at least one projection which is absorbable or partially absorbable and which is designed to take up at least half the implant's own weight of body fluids. Preferably, the projection is designed for insertion into an abdominal wall defect (e.g. the orifice of a hernia) and preferably withstands an overpressure in the abdominal cavity of more than 90 mbar. The protrusions are located towards the centre of the implant and would not be suitable for peripheral fixation of the implant.

FR2778544 A describes a mesh folded into a plug in which the folds are produced by drawstrings.

FR-A-2914179 relates to an implantable web intended for the reinforcement and support of biological tissues which has a structure comprising a "rigid" area and a "flexible" area. The flexible zone comprises one or more alteration means which create local structural heterogeneities in the base structure so as to reduce the rigidity of the base structure.

There is still a need for a surgical (in particular hernia repair) implant that is easy for a surgeon to implant and that has:
- Less material (i.e., less foreign bodies) and
- Greater assurance of tissue ingrowth on the parietal side and adhesion resistance on the visceral side, without implant buckling,
which implant is easy to fasten to bodily tissue.

It has now been found that these needs are overcome with the surgical implant according to claim 1. The implant comprises a first (ultimately parietal) and a second (ultimately visceral) layer. The first layer has at least two openings that are located in its peripheral area, and that are sized and shaped to allow for surgical fastening of the medical implant to bodily tissue.

In the following, some aspects of the invention are further disclosed in general terms.

The invention relates to a surgical implant for repair of a tissue or muscle wall defect comprising:
- a first layer, the first layer having a central area and a peripheral area, and
- a second layer
wherein
- the first layer has at least two openings located in the peripheral area of the first layer,
- the at least two openings are spaced apart from the periphery of the first layer,
- the surgical implant further has one or more raised sections wherein at least one of the one or more raised sections is in the first layer and wherein at least one of the one or more raised sections is in the peripheral area of the first layer,
- the at least two openings located in the peripheral area of the first layer are in the raised sections of the first layer,
- a part of each of the at least two openings is raised to a height of more than three times the thickness of the first layer in relation to the plane of the first layer, and
- the at least two openings are sized and shaped to allow for surgical fastening of the implant to bodily tissue,
wherein the at least two openings have maximum dimensions of 2 mm to 2 cm.

### Openings

According to the present invention, and in contrast to WO2009/049910 A1, the at least two openings are spaced apartfrom the edges of the first layer, so that the material in the periphery of the first layer adjacent the opening is intact. In other words, the at least two openings do not extend beyond the edge to include (or extend beyond) the periphery of the first layer. Also, a simple substantially linear incision in the plane of the first layer does not suffice because this would not result in that a part of each of the at least two openings is raised to a certain height in relation to the plane of the first layer. A simple substantially linear incision in the plane of the first layer would consequently not allow for surgical fastening of the implant to bodily tissue by virtue of the at least two openings.

As mentioned above, it is a requirement according to the invention that a part of each of the at least two openings is raised to a certain height in relation to the plane of the first layer. This height is more than three times the thickness of the first layer.

Moreover, it is preferred that the surgical implant further comprises a third layer facing the first layer opposite to the second layer being a synthetic absorbable film layer having openings, wherein the third layer preferably has a thickness of 2 µm to 60 µm.

Because typical surgical fastening (e.g. stapling or tacking) devices have a diameter of 2 to 8 mm, the openings have maximum dimensions of 2 mm to 2 cm and are capable to accept the entering of a surgical stapling or tacking instrument. Preferred are maximum dimensions in the range 4 mm to 2 cm. Alternatively, the openings are sized such that they are capable to accept the entering of a surgical needle; in this alternative they preferably have maximum dimensions of 2 mm to 8 mm.

The openings can be incisions (cut-ins), or can alternatively include the removal of material (cut-outs), or can alternatively be textile pores. Cutting can be achieved by mechanical means, or be an energy-based cutting; examples are knife cutting, laser cutting, radio frequency cutting, plasma cutting, or ultrasonic cutting. Moreover, textile pores can be buttonholes that are generally generated by sewing machines. Raw edges of a buttonhole are usually finished with stitching. The stitching of a buttonhole is preferably made from a resorbable polymer fibre. The stitching may have a different colour than the material of the first layer, so as to visualize the opening. As a further alternative, different pore sizes can be created also by textile means, e.g. a mesh-like structure can be prepared by sewing or embroidery. Bigger pores can be prepared in certain areas.

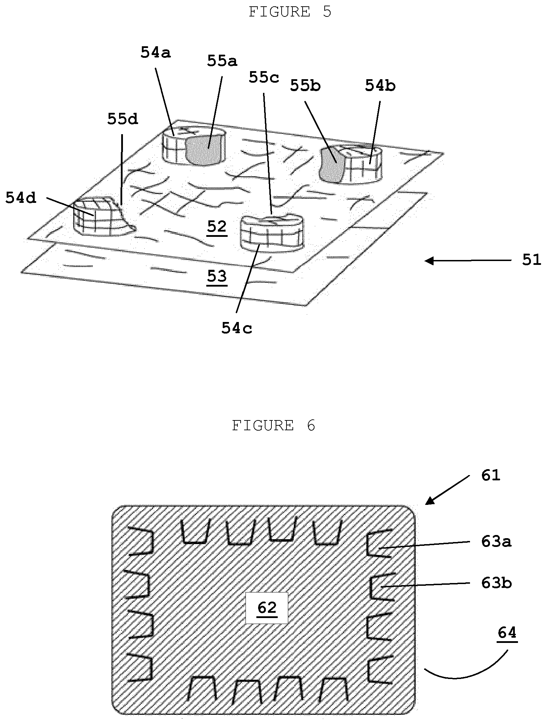

Moreover, it is preferred that at least some of the openings are provided as wings of the first layer, as they are generated by e.g. cut-ins (incisions) that are other than linear, see Fig. 6 below.

Furthermore, it is possible that at least one of the at least two openings is visually differentiated from other parts of the first layer (material). For instance, an opening may have a different colour than other parts of the first layer (material). This allows the surgeon an easier identification of the openings, when fastening the implant via the first (parietal) layer to bodily tissue.

Further, it is preferred that the at least two openings are arranged such that the fastening of the implant can be made by the surgeon in an open procedure via the centre area of the first layer, i.e. the openings are preferably accessible through the first layer's centre area.

### Raised sections

The implant further has one or more raised sections. At least one of the one or more raised sections is in the first layer. In a preferred alternative, the second layer has one or more raised sections. It is most preferred that both the first layer and the second layer each have one or more raised sections. Also, it is preferred that there are raised sections within the central area of the first layer.

The raised sections may be exemplified by embossed sections of a textile material. One embodiment of an embossed textile may be defined as a flattish textile structure consisting of filaments, with protrusions on parts of the surface. No additional material is necessary to create a raised section. Raised sections can be grouped in a more or less regular pattern, or alternatively at random. They may cover the implant completely or only partly.

The raised sections can be connected to each other directly or via areas between them with a base structure e.g. by thermal bonding, sewing, gluing, by fixing under pressure and also by simple contact.

Furthermore, a preferred implant has at least one of the one or more raised sections within the first layer being connected to each other directly or via areas between them with the second layer. Preferably, the raised sections are palpable and impart increased frictional engagement with tissue. As disclosed in WO99/45860 A1 and WO03/099160 A1, raised sections when on the visceral face of an implant advantageously prevent ingrowth of visceral tissue into the second (visceral) layer.

Depending on the arrangement and nature of the raised sections, they can also lead to increased rigidity. Raised sections may be varied in size, shape, and arrangement to impart desired elasticity-related properties, alter frictional engagement with tissue, and may serve as an orientation aid. In addition, material thickness may be varied for similar effects. The second and ultimately visceral layer, when having raised sections, preferably has a stretch elasticity that is higher than or the same as the stretch elasticity of the first and ultimately parietal layer.

The embossment to create raised sections is preferably in the millimetre range (0.5 mm to 10 mm). A typical thickness of the first layer is in the micron range (50 µm to 500 µm).

At least one of the one or more raised sections is in the peripheral area of the first layer. The at least two openings located in the peripheral area of the first layer are within a raised section of the first layer. It is preferred that at least one of the at least two openings located in the peripheral area of the first layer within a raised section of the first layer is a cutaway in some area or of some part of the raised section of the first layer.

The openings can be generated before embossment or after embossment.

### Possible shapes of the raised sections

The raised section can have the shape of a hump (protruded circular, oval, rounded rectangle, cross-like, cuboid, cylindrical, or pyramidal). Moreover, the embossment can be straight up, or oriented (askew), or conical with a base that is larger than the tip. A circular or linear elongated (corrugated) embossment will impart additional stiffness to the embossed layer. Moreover, the areal arrangement of the raised sections is preferably such that there are multiple protrusions derived from rings, ellipsoids, rounded rectangles, rectangles, or waves.

With respect to the shape or form of the raised sections, they preferably have a base area which is of a round, triangular, four-sided, pentagonal, hexagonal, symmetrical, asymmetrical, circular, elliptical, oval, square, rectangular, trapezoidal, or rhomboidal form, and a head area that is preferably of flat, pointed, round, concave, or convex form.

The at least two openings located in the peripheral area of the first layer is (are) within a raised section of the first layer. It is preferred that, additionally, the second layer has one or more raised sections.

It is thus most preferred that at least one of the at least two openings located in the peripheral area of the first layer is within a raised section of the first layer, and that the second layer also has one or more raised sections.

In accordance with the invention, the openings (in a raised section) are used as attachment points. For instance, intraperitoneal meshes can be sewn into the raised sections from the back, which minimises the risk of damaging underlying organs. A further option is for the raised section to have cutaways suitable to accommodate the end of a surgical instrument such as a stapler or tack applier, to allow the first layer of the implant to be attached to the stomach wall from the back in an open surgical procedure (e.g. a laparoscopic procedure).

Furthermore, it is preferred that the first and/or the second layer is (are) at least partially made from a surgical mesh, band, cord, film ribbon, woven structure, knitted material, fabric, and/or fleece, wherein the second layer preferably comprises a mesh, fleece, film, a woven structure, non-woven structure, a film, or a perforated film

### Material for the first layer

The first and ultimately parietal (tissue repair) layer is preferably made from a long term absorbable or permanent polymer fabric having pores of at least 1 mm diameter. These pores run through the first layer, and preferably run through the entire surgical implant.

A mesh-like material for the first layer is preferably macroporous with typical pore dimensions of greater than 0.5 mm, which supports good tissue integration. However, other pore sizes are also possible. As indicated above, a mesh or mesh-like material for the first layer can be provided in any kind known in the art, e.g., warp-knitted or weft-knitted or crochet-knitted or woven. Any filaments of the mesh may be bioabsorbable or non-absorbable, depending on the material. Thus, the mesh can be absorbable (resorbable), non-absorbable or partially absorbable. The filaments can be designed as mono-filaments or as multi-filaments. Tape yarns and drawn film tapes are possible as well. Any blends, mixtures or composites of materials and designs are also possible. Moreover, the filaments can be coated. Generally, the mesh-like layer is flexible and has an areal basic shape. For example, it can be based on a commercially available hernia repair mesh.

Suitable textile materials for the first layer are also well known in the art. Non-resorbable or very slowly resorbable substances include, e.g., polyalkenes (e.g. polypropylene or polyethylene), fluorinated polyolefins (e.g. polytetrafluoroethylene (PTFE) or polyvinylidene fluoride), polyamides, polyurethanes, polyisoprenes, polystyrenes, polysilicones, polycarbonates, polyarylether ketones (PEEKs), polymethacrylic acid esters, polyacrylic acid esters, aromatic polyesters, polyimides as well as mixtures and/or co-polymers of these substances. Other advantageous materials, many of them being resorbable, include polyhydroxy acids, polylactides, polyglycolides, polyhydroxybutyrates, polyhydroxyvaleriates, polycaprolactones, polydioxanones, poly-p-dioxanone, synthetic and natural oligo- and polyamino acids, polyphosphazenes, polyanhydrides, polyorthoesters, polyphosphates, polyphosphonates, polyalcohols, polysaccharides, polyethers, cellulose, bacterial cellulose, polyamides, aliphatic polyesters, aromatic polyesters, copolymers of polymerizable substances thereof, resorbable glasses. Particularly advantageous materials include polypropylene (non-resorbable), blends of polyvinylidene fluoride and copolymers of vinylidene fluoride and hexa-fluoropropene (non-resorbable, e.g. Pronova^{™} of Johnson & Johnson Medical GmbH) PTFE (non-resorbable; including ePTFE and cPTFE), polysilicones (non-resorbable), poly-p-dioxanone (PDS^{™}, resorbable), copolymers of glycolide and lactide (resorbable), in particular copolymers of glycolide and lactide in the ratio 90 : 10 (Vicryl^{™}, resorbable), copolymers of glycolide and ε-caprolactone (resorbable Monocryl^{™}). Biologic materials such as allografts and xenografts are possible as well.

Commercially available layers having raised sections are a mesh with rectangular bosses (e.g. VICRYL^{™}), a material with round bosses (e.g. SURGICEL^{™}) and a mesh (e.g. PROCEED^{™}, again all products of Ethicon).

### Material of the second layer

The second layer may be an anti-adhesive layer, to prevent or minimize adhesion to internal body structures such as bowel, liver or spleen to the implant. Suitable films or fabrics are from a non-resorbable material: PTFE sheet, fluorinated polyolefine (PVDF), copolymers of vinylidene fluoride and hexa-fluoropropene, silicone, durable polyvinyl alcohol gels like MiMedx's HydroFix Vaso Shield, polyurethane. Suitable resorbable materials are for example, poly-p-dioxanone (PDS^{™}), copolymers of glycolide and ε-caprolactone (e.g., Monocryl^{™} of Ethicon Inc) and/or copolymers of glycolide and lactide (in particular in the ratio 90:10, Vicryl^{™} of Ethicon Inc). Generally, a large variety of synthetic bioabsorbable polymer materials can be used, for example polyhydroxy acids (e.g., polylactides, polyglycolides, polyhydroxybutyrates, polyhydroxyvaleriates), polycaprolactones, polydioxanones, and PEG- or PEO-esters therof such as PLGA-PEG-PLGA or Methoxypoly-ethyleneglycol-PLGA, synthetic (but also natural) oligo- and polyamino acids, polyphosphazenes, polyanhydrides, polyorthoesters, polyphosphates, polyphosphonates, polyalcohols, polysaccharides, polyethers, polycyanoacrylates (Poly 2-OCA - co-BLCA) as cured from Ethicon's Omnex. However, naturally occurring materials such as fibrin, albumin, collagens and gelatine, hyaluronic acid like in Sanofi's Seprafilm or naturally derived materials such as bioabsorbable gel films or gel forming films, cross-linked omega 3-fatty acids like in Atrium's C-Qur mesh or oxygenized regenerated cellulose (ORC), crosslinked albumines or rh albumines like a cured material prepared from Progel^{®} Platinum Sealant or Progel^{®} AB both from Neomend or from Cryolife's BioFoam^{®}, where an albumin solution is cross-linked and foamed/expanded, crosslinked products prepared from Covidien's Duraseal where polyethylene glycol (PEG) ester solution and a trilysine amine are crosslinked are possible as well.

At least in part the second layer preferably comprises swelling or gel forming substances. The substances include surfactants such as PPO-PEO block copolymers (Pluronics), polysorbates such as polysorbate 20, 40, 60, 65, 80 (Tweens), or spans like Span 20 (sorbitan monolaurate), Span 40 (sorbitan monopalmitate), Span 60 (sorbitan monostearate), Span 65 (sorbitan tristearate), Span 80 (sorbitan monooleate), phospholipids, hydophilic natural or synthetic polymers such as alginate, dextrane, chitosane, carrageen, polyethylene glycol (PEG), soluble polyvinylalcohol (PVA), polyvinylpyrrolidone (PVP), carboxymethyl cellulose (CMC), and HES (hydroxyethyl starch). Hydrogel forming polymers may be obtained upon the polymerization or polyaddition or polycondensation containing at least one of the substances selected from the following group: polymerized hydroxyethyl methacrylate (HEMA); polymerized hydroxypropyl methacrylate (HPMA); polymerized a-methacryloyl-o-methoxy polyethylene glycol; polymerized methacryloyloxyethyl phosphorylcholine (MPC), polyethylene glycol-bisacrylate and copolymers thereof; cured resorbable prepolymers of type A-B-C-B-A with commercial examples sold a Focalseal^{®} (Genzyme) or Advaseal^{®} (Ethicon) with A = acryl or methacryl groups, B = hydrolytically splittable and containing polymers of lactide, glycolide, 2-hydroxybutyric acid, 2-hydroxyvaleriac acid, trimethylene carbonate, polyorthoesters, polyanhydrides, polyphosphates, polyphosphazenes and/or polyamides and/or copolymers thereof, and C = hydrophilic polymers, in particular polyethylene glycol (PEG), polyvinyl alcohol (PVA), polyvinyl pyrrolidone (PVP), poly-N-isopropylacrylamide (PNiPAAM). The following examples are illustrative of the principles and practice of the present invention although not limited thereto.

With regard to the first and second layers, they are preferably at least partially made from a surgical mesh, band, cord, film ribbon, woven structure, knitted material, fabric, and/or fleece.

### Connection between the first and second layer

Preferably, the second layer is at least partially attached to the first face of the first layer, optionally with one or more layers in-between the first (and ultimately parietal) and second (ultimately visceral) layer. The first (preferably embossed fabric) tissue repair layer and the second layer can be connected to each other, in any way, e.g., sewn, embroidered, bonded (including by thermal means), or welded thermally including ultrasonically. The welding techniques also include, in a broader sense, thermal deformation of at least one of the layers (below the melting point of the layer). An absorbable melt glue such as polydioxanone as a relatively low melting bioabsorbable polymer might be used as a gluing member for the first and second layer material. Other soluble polymers such as polylactide, polycaprolactone or copolymers thereof might be used as solvent glues. Reactive glues like cyanoacrylates or isocyanates or oxiranes may also be used, if biocompatible.

In one aspect of the invention, the first and second layers are made in one piece as a spacer fabric, whereby anti-adhesive fibres make up the visceral side.

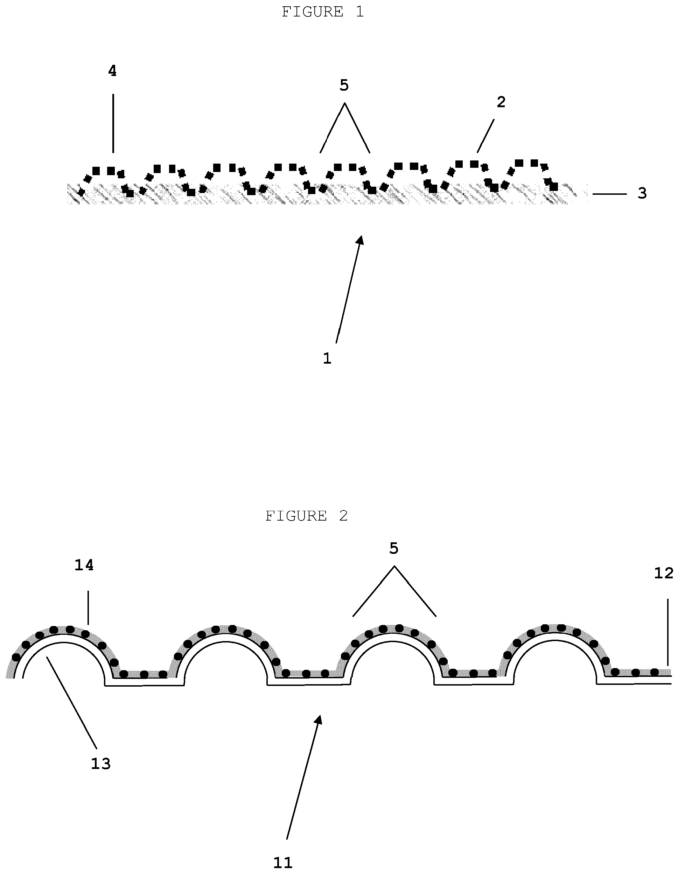

In one aspect of the invention the adhesion barrier film might be cast as a solution or melt in a certain thickness and the protruded first layer is placed on top. During solidifying by solvent evaporation or chilling down, the mesh will be partially embedded in the film and only protruded areas will be basically free of film (see Fig. 1). In one aspect the adhesion barrier is build by casting a precursor solution or liquid mixture in a mould, place the protruded first layer on top by partly embedding in the non-protruded regions and allow polymerize for a good connection for at least the first layer of fibres to be completely embedded. No additional mesh layer or particular fibre surface treatment, such as corona treatment, is needed to ensure sufficient attachment.

### Active ingredients

It may be advantageous to provide an implant of the present invention that has at least one biologically active or therapeutic ingredient which can optionally be released locally after the implantation. Substances which are suitable as active or therapeutic agents may be naturally occurring or synthetic, and include and are not limited to, for example, antibiotics, antimicrobials, antibacterials, antiseptics, chemotherapeutics, cytostatics, metastasis inhibitors, antidiabetics, antimycotics, gynecological agents, urological agents, anti-allergic agents, sexual hormones, sexual hormone inhibitors, haemostyptics, hormones, peptide-hormones, antidepressants, vitamins such as Vitamin C, antihistamines, naked DNA, plasmid DNA, cationic DNA complexes, RNA, cell constituents, vaccines, cells occurring naturally in the body or genetically modified cells. The active or therapeutic agent may be present in various forms including in an encapsulated form or in an adsorbed form. With such active agents, the patient outcome may be improved or a therapeutic effect may be provided (e.g., better wound healing, or inflammation inhibition or reduction).

One preferred class of active agents is antibiotics that include such agents as gentamicin or ZEVTERA^{®} (ceftobiprole medocaril) brand antibiotic (available from Basilea Pharmaceutica Ltd., Basel Switzerland). Other active agents that may be used are highly effective, broad-band antimicrobials against different bacteria and yeasts (even in the presence of bodily liquids) such as octenidine, octenidine dihydrochloride (available as active ingredient in Octenisept^{®} disinfectant from Schulke & Mayr, Norderstedt, Germany), polyhexamethylene biguanide (PHMB) (available as active ingredient in Lavasept^{®} from Braun, Switzerland), triclosan, copper (Cu), silver (Ag), nanosilver, gold (Au), selenium (Se), gallium (Ga), taurolidine, N-chlorotaurine, alcohol-based antiseptics such as Listerine(R) mouthwash, N a-lauryl-L-arginine ethyl ester (LAE), myristamidopropyl dimethylamine (MAPD, available as an active ingredient in SCHERCODINE^{®} M), oleamidopropyl dimethylamine (OAPD, available as an active ingredient in SCHERCODINE^{®} O), and stearamidopropyl dimethylamine (SAPD, available as an active ingredient in SCHERCODINE^{®} S), fatty acid monoesters, and most preferably octenidine dihydrochloride (hereinafter referred to as octenidine), Taurolidine, and PHMB.

One preferred class of active agents are local anesthetics that includes such agents as: Ambucaine, Benzocaine, Butacaine, Procaine/Benzocaine, Chloroprocaine, Cocaine, Cyclomethycaine, Dimethocaine/Larocaine, Etidocaine, Hydroxyprocaine, Hexylcaine, Isobucaine, Paraethoxycaine, Piperocaine, Procainamide, Propoxycaine, Procaine/Novocaine, Proparacaine, Tetracaine/Amethocaine, Lidocaine, Articaine, Bupivacaine, Dibucaine,Cinchocaine/Dibucaine, Etidocaine, Levobupivacaine, Lidocaine/Lignocaine, Mepivacaine, Metabutoxycaine, Piridocaine, Prilocaine, Propoxycaine, Pyrrocaine, Ropivacaine, Tetracaine, Trimecaine, Tolycaine, combinations thereof, e.g., Lidocaine/prilocaine (EMLA) or naturally derived local anesthetics including Saxitoxin, Tetrodotoxin, Menthol, Eugenol and pro-drugs or derivatives thereof.

Additionally, a contrast agent may be incorporated into the devices of the present invention. Such a contrast agent may be a gas or gas creating substance for ultrasound contrast or MRI contrast, such as metal complexes like GdDTPA or superparamagnetic nanoparticles (Resovist^{®} or Endorem^{®}) as taught in EP 1 324 783 B1. X-Ray visible substances might be included as shown in the EP 1 251 794 B1 including pure zirconium dioxide, stabilized zirconium dioxide, zirconium nitride, zirconium carbide, tantalum, tantalum pentoxide, barium sulphate, silver, silver iodide, gold, platinum, palladium, iridium, copper, ferric oxides, not very magnetic implant steels, non-magnetic implant steels, titanium, alkali iodides, iodated aromatics, iodated aliphatics, iodated oligomers, iodated polymers, alloys of substances thereof capable of being alloyed. The contrast agents may be included in or on a mesh first layer, or in or on the second adhesion barrier layer.

### Long-term stability

In a particularly advantageous embodiment, the surgical implant according to the invention comprises a first layer designed as a long-term stable soft-tissue repair mesh comprising pores having a size of at least 1 mm.

In one embodiment, the surgical mesh (Fig. 1) is preferably a long-term stable base component that may be non-absorbable or slowly absorbable. As used herein, the terminology long-term, stable base component means a non-resorbable polymer or a very slowly resorbable polymer that desirably possess at least 50 percent of its original tearing strength 60 days after implantation. In one embodiment, the long-term, stable base component preferably includes substances such as polyamides, which are generally regarded as resistant and non-resorbable materials, and which may be exposed over time to body tissue and tissue fluids.

In one embodiment, the surgical mesh (Fig. 1) may be made of one or more materials including polypropylene, mixtures of polyvinylidene fluoride, and/or copolymers of vinylidene fluoride, hexafluoropropene, polyglycolide-polylactide, or polyglecaprone (i.e., MONOCRYL). In one embodiment, the surgical mesh 40 may be made from monofilaments, multifilament and/or threads having different diameters/sizes. In one embodiment, the surgical mesh 40 is desirably warp knitted.

In one embodiment, the surgical mesh preferably includes non-resorbable threads of polypropylene having a diameter of between about 0.089 to 0.13 mm, polyvinylidene fluoride copolymer threads with a diameter of about 0.069 to 0.089 mm, PVDF threads with a diameter of about 0.089 to 0.13 mm, polyester threads with a diameter of about 0.08 to 0.12 mm and/or polyamide threads with a diameter of about 0.010 to 0.13 mm.

Depending upon the intended use of the tissue repair device, a biocompatible long-term-stable polymer may be used to manufacture the fabric repair member. By a long-term-stable polymer is meant a non-resorbable biocompatible polymer, or a bioabsorbable polymer which absorbs or degrades slowly, for example which possesses at least 50% of its original tearing strength in vivo 60 days after implantation. The latter group includes substances such as polyamides, which generally are regarded as resistant, as they are not designed as resorbable materials, but are attacked over time by body tissue and tissue fluids. Preferred materials for the fabric repair member include polyhydroxy acids, polylactides, polyglycolides, polyhydroxy butyrates, polyhydroxy valeriates, polycaprolactones, polydioxanones, synthetic and natural oligo- and polyamino acids, polyphosphazenes, polyanhydrides, polyorthoesters, polyphosphates, polyphosphonates, polyalcohols, polysaccharides, polyethers, polyamides, aliphatic polyesters, aromatic polyesters, copolymers of polymerizable substances thereof, resorbable glasses.

### Examples for applications of the medical implant

Soft tissue repair implants such as surgical meshes are mainly used when a defect or weakness exists in soft tissue or a tissue hole has to be filled or covered:
(a) Ventral and inguinal hernias occur when a tissue, a structure, or part of an organ protrudes through an abnormal opening in the body. It is most commonly associated with the projection of the intestine through a weak point in the abdominal wall. Hernia repair devices could be made in different shapes and from different materials, in the form of flat devices, basically flat but curved devices, pouches, bags or folded into plugs.
(b) Surgical meshes, tapes or slings are used in the field of pelvic disorders like stress urinary incontinence or pelvic organ prolapse. In these applications, there may be a need to place the fabric in contact with the vaginal wall (e.g., a pelvic mesh) or in contact with the urethra such as with the GYNECARE^{®} TVT system from Ethicon, Inc., wherein the inventive assembly might support the locking fixation in certain regions of the tape or mesh.
(c) Durapatches are used after brain surgery to cover and close the *dura mata.* The *dura mata* is the tough, inflexible fibrous sheath which is the outermost of three layers that surround the brain and spinal cord. Commercial grafts are made up of either biologic (this includes xenografts and allografts) or synthetic material. The inventive protruded patches in certain areas on one of the both sides might help to keep the implant in place and could facilitate fixation.
(d) Rotator cuff reinforcement grafts are most often used in cases where existing tissue can no longer be used or treated to support the rotator cuffs functions.
(e) Absorbable pouches are used in the field of trauma surgery as a liver compression device to reduce bleeding, like Vicryl° Mesh bag A".
(f) Grafts in the field of breast reconstruction are used with the "TRAM-flap" procedure, where an autogeneous tissue reconstruction of the breast is performed with the transverse *rectus abdominis myocutaneous* (TRAM) flap from the chest. The abdominal wall donor site for the muscle flap might develop a potential abdominal wall weakness, bulging, and hernia. To prevent hernia, most surgeons will use a synthetic mesh when closing the abdominal wall. Fabrics such as absorbable meshes like Vicryl^{®} mesh or TiGr^{®} matrix are also used in breast augmentation or reconstruction i.e. in oncoplastic surgery which is defined as a combination of tumour excision, with appropriate margin including lumpectomy or mastectomy, and immediate reconstruction of the breast (Koo et al. 2011 "Results from Over One Year of Follow-Up for Absorbable Mesh Insertion in Partial Mastectomy", Yonsei Med J 52(5):803-808, 2011). The inventive devices help to minimize sutures, tacks or glues to facilitate fixation.
(g) Soft tissue repair devices are used as a filler, to bulk tissue e.g. in cosmetic surgery to remove wrinkles or in fistula surgery to fill the fistula channels. Depending on the intended use, absorbable materials might be used.

The implant may be made of one or more of the materials selected from the group of: polyalkenes, polypropylene, polyethylene, fluorinated polyolefins, polytetrafluorethylene, polyvinylidene fluoride, polyamides, polyurethanes, polyisoprenes, polystyrenes, polysilicons, polycarbonates, polyarylether ketones, polymethacryl acid esters, polyacryl acid esters, aromatic polyesters, polyimides, polyhydroxy acids, polylactides, polyglycolides, polyhydroxybutyrates, polyhydroxy valemates, polycaprolactones, polydioxanones, synthetic and natural oligo and polyamino acids and their derivatives and poly (y-benzyl-L-glutamates), pseudopolyamino acids, proteins, collagens, polyphosphazenes, polyanhydrides, polyorthoesters, polyoxaesters, polyphosphates, polyphosphonates, polyalcohols, polysaccharides, dextrans, dextran sulfates, chitosans, starches, hydroxyethyl starches, hydroxypropyl starches, oxygenated regenerated cellulose, hyaluron acids, polyethers, polyethylene glycols, poly(ethylene glycols-copropylene glycols), polycyanoacrylates, polyvinylpyrrol-idones, mixtures of such substances, copolymers of such substances.

In a second aspect, the disclosure provides a process of fabricating the raised sections in the implant of the invention, which process comprises the steps of
- connecting a first layer and a second layer, which attaching is preferably by gluing or bonding, wherein the first layer is preferably a surgical mesh and the second layer is preferably a resorbable polymer film;
- forming one or more raised sections within the first layer or the second layer, preferably within both the first and the second layers, by either:
   - pressing and thermally deforming the first layer attached to the second layer, or
   - drawing-out of the first layer as attached to the second layer.

Furthermore, the disclosure provides a process for fabricating the implant according to the invention, comprising the steps of (a) connecting a first and a second layer by gluing, laminating, stitching or embroidering; and (b) thermoforming the first and second layers by applying heat and pressure.

In a further alternative embodiment, the disclosure provides a process for fabricating the implant according to the invention comprising the steps of (a) thermoforming a surgical mesh to generate multiple protrusions; (b) placing the thermoformed mesh in substantially flat mould together with a liquid composition for a second layer material; and (c) solidifying the second layer material by solvent evaporation, curing, reacting, cross-linking, or chilling down.

In a third aspect, the disclosure provides a method of implanting the above-described implant by using an open intraperitoneal onlay technique where the raised sections face the abdominal wall and the parietal facing layer is sewn to the abdominal wall through the openings in the raised sections, minimizing the risk to damage body structures below with the needle.

In the following, the invention is further explained by means of embodiments. The drawings show in
Figure 1 a first embodiment of the medical implant of the invention as seen from the side;
Figure 2 an alternative embodiment of the medical implant, again seen from the side, wherein the openings are not shown;
Figure 3 photographs of an embossed first layer (Fig. 3a - parietal face, Fig. 3b - visceral face), which could be trimmed to the periphery of the protrusion, again, the openings are not shown;
Figure 4 sketches of a thermo-mould for fabricating raised sections in the implant;
Figure 5 the implant of the invention in an alternative embodiment, as seen from the side, wherein raised sections are provided in the first layer, which raised sections have cutaways;
Figure 6 the medical implant as seen from the top (view onto the first layer), with the embodiment having wings;
Figure 7 a surgical implant according to the invention wherein a single raised section within the first layer forms a rim, and 7b illustrating cut-ins in the raised section (rim); and
Figure 8 a 3D view of the first layer having raised sections.

As shown in Fig. 1, implant (1) may be prepared from adhesion barrier film (3). When producing implant (1), film (3) is cast as a solution (or melt) in a certain thickness. Protruded first mesh layer (2) is placed on top. During solidifying by solvent evaporation or chilling down, mesh (2) is partially embedded into film (3) and only protruded areas or fibres (4) will basically be free from film (3). No additional mesh layer or particular fibre surface (e.g. corona) treatment is needed to ensure sufficient attachment of first mesh layer (2) to second layer (3). A raised section is shown as (5). Protrusions are over the whole implant area and allow both improved peripheral as well as improved central fixation to pull fixate for examples to the margin of a hernia defect, minimizing the risk to penetrate underlying tissues or organs when using a stapling or tacking instrument, or a surgical needle.

As shown in Fig. 2 (cf. also Example 1 below), in an implant (11), a mesh (14) can be more or less fully embedded in a thin PDS film (12). PDS film (12) with embedded mesh (14) thus forms the first layer, which first layer is laminated to a second (e.g. ORC) layer (13). A raised section is shown as (15) .

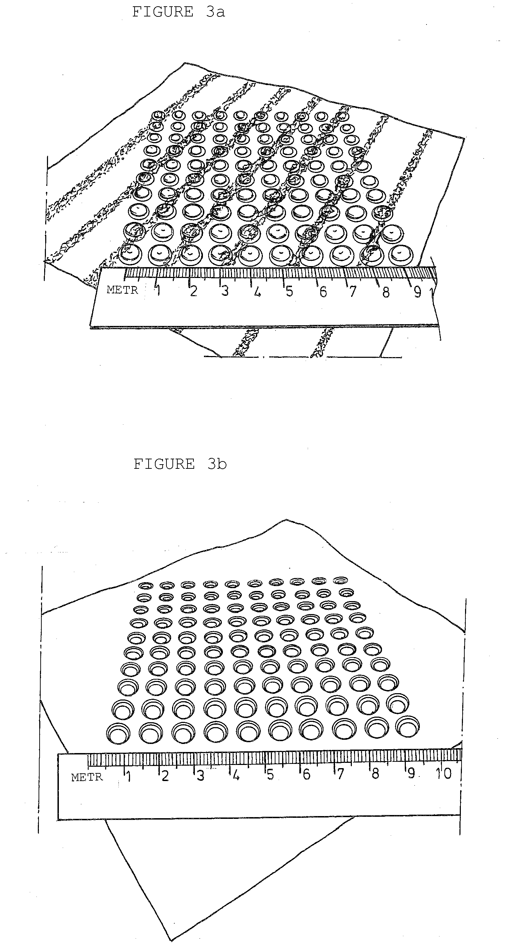

As shown in Fig. 3, and with reference to Example 1 below, an ORC layer when used as a first layer can be embossed. The view onto the first and ultimately parietal layer is shown in Fig. 3a, and the view onto the second layer is shown in Fig. 3b.

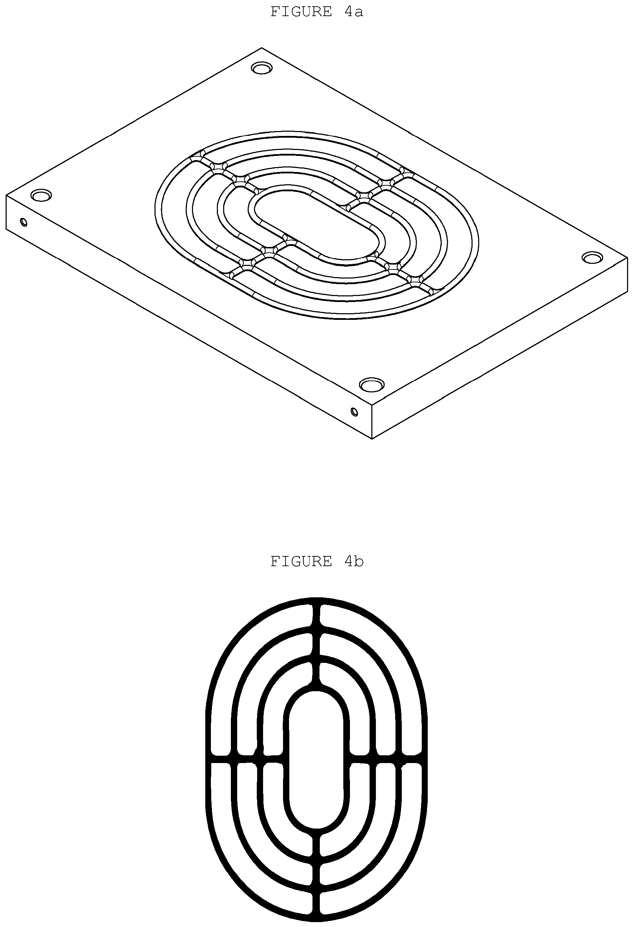

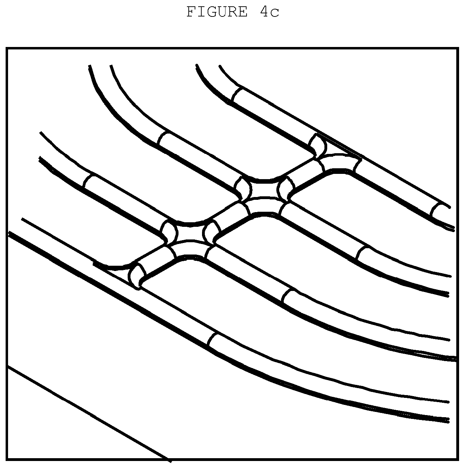

Fig. 4a shows a thermo-mould for creating raised sections. Corresponding Fig. 4b shows a substantially identical pattern but with one additional rim-shaped raised section. Fig. 4c then shows an enlarged view onto a part of Fig. 4a: the pattern that is suitable to create raised sections is 3 mm wide at the bottom, it has straight walls having a height of 0.5 mm, and the top is a half circle (d = 3 mm).

Fig. 5 shows an alternative embodiment of the implant of the invention (51), as seen from the side, having a first (parietal) layer (52). Implant (51) also has a second layer (53). Raised sections (54a-d) as provided in the peripheral area of the first layer contain cutaways (55a-d) that are suitable to accommodate the end of a surgical instrument, so that implant (51) can be fastened to bodily tissue through cutaways (55a-d) of the first layer (52) of implant (51).

Fig. 6 illustrates a top view of implant (61) with the first layer (62) on top, the first layer having twelve wings (63a,63b etc.) as cut-ins in its peripheral area. First layer (62) is connected to second layer (64), such as commercially available (i) MONOCRYL^{®} or (ii) INTERCEED^{®}, or (iii) PDS + IN-TERCEED^{®} (all products of Ethicon) film. Wings (63a,63b etc.) may alternatively have a variety of shapes (e.g. rectangular, triangular, semi-circular). In addition, one, several or all wings (63a,63b etc.) may extend to overlap the surface, i.e. while the wings may be simple cut-ins in the first layer, they may alternatively contain some additional first layer material and extend to overlap the material of the first layer.

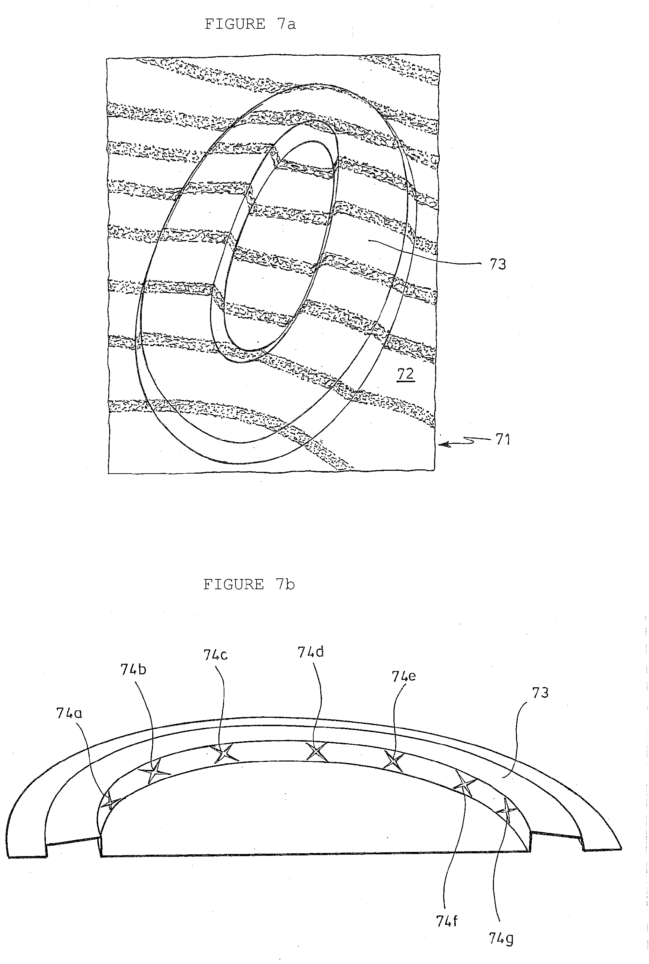

Fig. 7 shows surgical implant (71) according to the invention wherein a raised section within the first layer (72) forms one rim (73). As shown in Fig. 7b, openings (74a-g) are located within rim (73) and are marked "X"; they are provided as crossing cuts and accommodate for a fastening device when surgically implanting said implant (71).

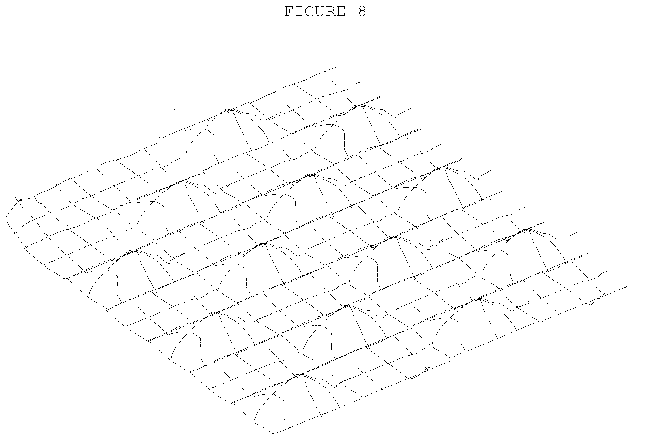

Fig. 8 is a 3D sketch of a first layer having raised sections that would accommodate cutaways as openings.

The following examples serve to illustrate, but not to limit the present invention.

### Examples

**Modified Proceed mesh** is an experimental complex laminate derived from commercial Proceed^{®} mesh (Ethicon Inc.) having an ORC (oxygenized regenerized cellulose) layer (visceral side) followed by a thin polydioxanone film having 2 mm diameter perforations 2 mm spaced apart over the whole mesh, polypropylene mesh, a thin polydioxanone film;
**Dynamesh^{®} Ipom** (by FEG) is a dual-faced mesh having polypropylene fibres on the first side and PVDF (polyvinylidene fluoride) fibres on the second side (visceral side). The pore size is larger than 1 mm; and
**Physiomesh^{®}** is an intraperitoneal mesh implant having a large pored polypropylene mesh laminated between two resorbable Monocryl^{®} films.

### Example 1) Modified Proceed mesh with one hundred 3 mm embossments on the parietal side

A Proceed^{®} mesh (15 x 15 cm) was placed in a boss mould that had 10 x 10 cylindrical protrusions of d = 5 mm, h = 5 mm. The distance of the protrusions from each other was about 10 mm (centre to centre). The counterpart cylindrical drill holes of d = 7 mm, depth = 10 mm were also 10 mm apart from one another. The assembled mould was heated on a hotplate at 120°C on each side for about 1 minute without pressure. The mould was then placed in an unheated press and slowly pressed using a hand wheel, and mesh and mould were cooled for about 30 mins. The mesh thus had 100 bosses of rounded conical shape and of a height of about 3 mm that pointed in the direction of the PDS side. Because of the bosses it was possible to sew only the bosses with a Prolene^{™} 2-0 thread, minimizing the risk to capture or punch the bowel with the needle during suturing. The resulting material is an example of the implant according to the invention and is shown in Fig. 3. However, Fig. 3 does not show the openings.

### Example 2) Dynamesh^{®} IPOM -Parietal Side embossed

A rectangle of 20 x 22 cm was cut from a commercial Dynamesh^{™} and placed into a metal thermo-mould made out of 2 parts (positive and negative, see Fig. 4a to 4c below). On the mould, four rounded concentric rectangles protruded with a bottom strut width of 3 mm (radius of 1,5 mm and high of 2 mm). The dimensions of the rounded rectangles were as follows:
(1) length 16.5 cm, width 12.5 cm, radius 6.25 cm,
(2) l = 13.5 cm, w = 9.5 cm, r= 4.75 cm,
(3) l = 10.5 cm, w = 6.5 cm, r = 3.25 cm,
(4) l = 7.5 cm, w = 3.5 cm, r = 1.75 cm.

Dimensions were measured from the tip of the protrusions.

The rounded rectangle protrusions with a total high of about 2 mm, a width on the foot section of 3 mm and a radius of 1.5 mm on the tip section) were connected by two horizontal and two vertical struts of 3 mm width and the same height. The intersections of embossed circles and struts were slightly rounded, to prevent cutting or rupturing the mesh during thermoforming. The negative had a width of about 4 mm.

The mesh was heated in a heat press between the two parts of the mould to 110°C, at about 10 bar, and cooled under pressure.

The mesh showed visible, palpable protrusions on the parietal side with a height of about 1 to 1.5 mm, which made side differentiation easy and which could easily and a gripping with a needle and 2-0 Prolene^{®} suture easy in the rip area, minimizing the risk of potentially piercing the intestine during an operation.

### Example 3) Ultrapro^{®} mesh with 4 bosses + Monocryl^{®} film

A 20 x 20 cm square Ultrapro^{®} mesh (Ethicon) was thermally deformed at all four corners. The high grade steel mould had a cylindrical raised area 8 mm high with a diameter of 18 mm and a corresponding counterpart. After 2 minutes at 110°C in a hot press, the resulting bosses had a height of about 5 mm and a diameter of about 18 mm and were easy to see and could also be felt through a latex glove. Then about a third of each boss was cut away from the top to the base facing to the centre. This created stronger resistance to a finger sliding over them against the cut than with the cut. In addition, cutting the raised sections in this way is useful for making positioning easy by pushing a finger into them. The bosses could be easily entered with a stapler. The thermoformed mesh was attached to a 50 µm Monocryl^{®} film by embroidery circular in the centre region and circumferential outside the embossed area. See the sketch in Fig. 5.

### EXAMPLE 5) Physiomesh^{®} with elevatable peripheral wings

A Physiomesh^{®} layer was provided with U-shaped wings having a length of 3 cm and a width of about 2 cm. The wings were created as cut-ins using a laser cutter (CAD CAM). The flaps face towards the centre and start at least 2 cm from the edge. The wing could be in plane, liftable or slightly out of plane to facilitate the lifting and entry of devices like a hernia stapler. See the sketch in Fig. 6.

### Example 6) Prolene Soft^{™} mesh laminated between two thin PDS^{®} films having multiple peripheral cut-ins

A polypropylene mesh (Prolene Soft^{®} mesh) was heat-laminated between two polydioxanone films of each 5 µm and was thermoformed with a metal form and counter-form, whereby the positive form had the following profile (d = 3 cm flat centre section, followed on each end with a 1 cm embossed flank, linear descending rim section over about 2.5 cm to 0.5 cm high, and an almost vertical dropping zone to 0.5 cm). The 1 cm embossed region was cut-in circumferential with cross-cuts of about 1 cm, spaced apart about 1 cm. The cut-in could be easily penetrated by a stapling device, allowing stapling to the abdominal wall from the visceral side of the mesh. See Fig. 7a and the sketch in Fig. 7b.

## Claims

1. Surgical implant (1, 11, 51, 61, 71) for repair of a tissue or muscle wall defect comprising:
- a first layer (2, 12, 52, 62, 72), the first layer having a central area and a peripheral area, and
- a second layer (3, 13, 53, 64),
wherein
- the first layer has at least two openings (55, 63, 74) located in the peripheral area of the first layer,
- the at least two openings are spaced apart from the periphery of the first layer,
and **characterised in that**
- the surgical implant further has one or more raised sections (5, 15, 54) wherein at least one of the one or more raised sections is in the first layer and wherein at least one of the one or more raised sections is in the peripheral area of the first layer,
- the at least two openings located in the peripheral area of the first layer are in the raised sections of the first layer,
- a part of each of the at least two openings is raised to a height of more than three times the thickness of the first layer in relation to the plane of the first layer, and
- the at least two openings are sized and shaped to allow for surgical fastening of the implant to bodily tissue, wherein the at least two openings have maximum dimensions of 2 mm to 2 cm.

2. Implant according to claim 1, **characterized in that** it further comprises a third layer facing the first layer opposite to the second layer being a synthetic absorbable film layer having openings, wherein the third layer preferably has a thickness of 2 µm to 60 µm.

3. Implant according to any one of the preceding claims, **characterized in that** the at least two openings have maximum dimensions of 1 mm to 9 mm.

4. Implant according to any one of the preceding claims, **characterized in that** at least one of said at least two openings located in the peripheral area of the first layer within a raised section of the first layer is a cutaway (54) in some area or of some part of the raised section of the first layer.

5. Implant according to any one of the preceding claims, **characterized in that** at least one of the at least two openings is provided as a wing (63) within the first layer.

6. Implant according to any one of the preceding claims, **characterized in that** the second layer has one or more raised sections.

7. Implant according to any one of the preceding claims, **characterized in that** the first and/or the second layer is at least partially made from a surgical mesh, band, cord, film ribbon, woven structure, knitted material, fabric, and/or fleece, wherein the second layer preferably comprises a mesh, fleece, film, a woven structure, non-woven structure, a film, or a perforated film.

8. Implant according to any one of the preceding claims, **characterized in that** there are one or more raised sections within the central area of the first layer, preferably wherein said raised sections have a base area which is of a round, triangular, tetragonal, pentagonal, hexagonal, symmetrical, asymmetrical, circular, elliptical, oval, square, rectangular, trapezoidal, or rhomboidal form, and the head area is of flat, pointed, round, concave, or convex form.

9. Implant according to any one of the preceding claims, **characterized in that** at least one of the at least two openings is visually differentiated from other parts of the first layer.

10. Implant according to any one of claims 4 to 8, **characterized in that** at least some of the one or more raised sections within the first layer are connected to each other directly or via areas between them with the second layer.

11. Surgical implant according to any one of the preceding claims, **characterized in that** the first layer comprises at least one of the materials selected from the following list: polyalkenes, polypropylene, polyethylene, fluorinated polyolefins, polytetrafluoroethylene, PTFE, ePTFE, cPTFE, polyvinylidene fluoride, blends of polyvinylidene fluoride and copolymers of vinylidene fluoride and hexafluoropropene, polyamides, polyurethanes, polyisoprenes, polystyrenes, polysilicones, polycarbonates, polyarylether ketones, polymethacrylic acid esters, polyacrylic acid esters, aromatic polyesters, polyimides, polyhydroxy acids, polylactides, polyglycolides, copolymers of glycolide and lactide, copolymers of glycolide and lactide in the ratio 90:10, polyhydroxybutyrates, polyhydroxyvaleriates, polycaprolactones, copolymers of glyco¬l¬ide and ε-caprolactone, polydioxanones, poly-p-dioxanone, synthetic and natural oligo- and polyamino acids, poly-phosphazenes, polyanhydrides, polyorthoesters, polyphosphates, polyphosphonates, polyalcohols, polysaccharides, polyethers, polyamides, aliphatic polyesters, aromatic polyesters, polyurethanes, copolymers of polymerizable substances there¬of, resorbable glasses, cellulose, bacterial cellulose, allograft, xenograft, collagen, gelatin, and silk.

12. Surgical implant according to any one of the preceding claims, **characterized in that** the second layer comprises a material selected from the following list: synthetic bioabsorbable polymer materials, polyhydroxy acids, polylactides, polyglycolides, copolymers of glycolide and lactide, copolymers of glycolide and lactide in the ratio 90:10, polyhydroxybutyrates, polyhydroxyvaleriates, polycaprolactones, copolymers of glycolide and ε-caprolactone, polydioxanones, poly-p-dioxanone, synthetic and natural oligo- and polyamino acids, polyphosphazenes, polyanhydrides, polyorthoesters, polyphosphates, polyphosphonates, polyalcohols, polysaccharides, polyethers, collagen, gelatin, bioabsorbable gel films cross-linked with omega 3 fatty acids, and oxygenized regenerated cellulose, wherein said second layer optionally further comprises a material selected from the following list: synthetic non resorberble polymer materials, polyalkenes, polypropylene, polyethylene, fluorinated polyolefins, polytetrafluoroethylene, PTFE, ePTFE, cPTFE, polyvinylidene fluoride, blends of polyvinylidene fluoride and copolymers of vinylidene fluoride and hexafluoropropene, polyamides, polyurethanes, polyisoprenes, polystyrenes, and polysilicones.

## Patentansprüche

1. Chirurgisches Implantat (1, 11, 51, 61, 71) zur Reparatur eines Gewebe- oder Muskelwanddefekts, umfassend:
- eine erste Schicht (2, 12, 52, 62, 72), wobei die erste Schicht einen mittleren Bereich und einen Randbereich aufweist, und
- eine zweite Schicht (3, 13, 53, 64),
wobei
- die erste Schicht wenigstens zwei Öffnungen (55, 63, 74) aufweist, die in dem Randbereich der ersten Schicht angeordnet sind,
- die wenigstens zwei Öffnungen von dem Rand der ersten Schicht beabstandet sind,
und **dadurch gekennzeichnet, dass**
- das chirurgische Implantat ferner einen oder mehrere erhöhte Abschnitte (5, 15, 54) aufweist, wobei wenigstens einer des einen oder der mehreren erhöhten Abschnitte in der ersten Schicht angeordnet ist und wobei wenigstens einer des einen oder der mehreren erhöhten Abschnitte in dem Randbereich der ersten Schicht angeordnet ist,
- die wenigstens zwei Öffnungen, die in dem Randbereich der ersten Schicht angeordnet sind, in den erhöhten Abschnitten der ersten Schicht angeordnet sind,
- ein Teil von jeder der wenigstens zwei Öffnungen auf eine Höhe von mehr als dreimal der Dicke der ersten Schicht von der Ebene der ersten Schicht erhöht ist, und
- die wenigstens zwei Öffnungen bemessen und geformt sind, chirurgische Befestigung des Implantats an Körpergewebe zu ermöglichen, wobei die wenigstens zwei Öffnungen größte Abmessungen von 2 mm bis 2 cm aufweisen.

2. Implantat gemäß Anspruch 1, **dadurch gekennzeichnet, dass** es ferner eine dritte Schicht, die der ersten Schicht gegenüber der zweiten Schicht zugewandt ist, umfasst, die eine synthetische absorbierbare Filmschicht ist, die Öffnungen aufweist, wobei die dritte Schicht vorzugsweise eine Dicke von 2 µm bis 60 µm aufweist.

3. Implantat gemäß einem der vorstehenden Ansprüche, **dadurch gekennzeichnet, dass** die wenigstens zwei Öffnungen größte Abmessungen von 1 mm bis 9 mm aufweisen.

4. Implantat gemäß einem der vorstehenden Ansprüche, **dadurch gekennzeichnet, dass** wenigstens eine der wenigstens zwei Öffnungen, die in dem Randbereich der ersten Schicht innerhalb eines erhöhten Abschnitts der ersten Schicht angeordnet sind, ein Ausschnitt (54) in einem Bereich oder einem Teil des erhöhten Abschnitts der ersten Schicht ist.

5. Implantat gemäß einem der vorstehenden Ansprüche, **dadurch gekennzeichnet, dass** wenigstens eine der wenigstens zwei Öffnungen als ein Flügel (63) innerhalb der ersten Schicht bereitgestellt ist.

6. Implantat gemäß einem der vorstehenden Ansprüche, **dadurch gekennzeichnet, dass** die zweite Schicht einen oder mehrere erhöhte Abschnitte aufweist.

7. Implantat gemäß einem der vorstehenden Ansprüche, **dadurch gekennzeichnet, dass** die erste und/oder die zweite Schicht wenigstens zum Teil aus einem/einer chirurgischen Netz, Streifen, Schnur, Filmband, gewebten Struktur, gestrickten Material, Gewebe und/oder Vlies besteht, wobei die zweite Schicht vorzugsweise ein/einen Netz, Vlies, Film, gewebte Struktur, nichtgewebte Struktur, Film oder einen perforierten Film umfasst.

8. Implantat gemäß einem der vorstehenden Ansprüche, **dadurch gekennzeichnet, dass** ein oder mehrere erhöhte Abschnitte innerhalb des mittleren Bereichs der ersten Schicht vorliegen, wobei die erhöhten Abschnitte vorzugsweise eine Grundfläche aufweisen, die eine runde, dreieckige, viereckige, fünfeckige, sechseckige, symmetrische, asymmetrische, kreisförmige, elliptische, ovale, quadratische, rechteckige, trapezförmige oder rhombische Form aufweisen und der Kopfbereich eine flache, spitze, runde, konkave oder konvexe Form aufweist.

9. Implantat gemäß einem der vorstehenden Ansprüche, **dadurch gekennzeichnet, dass** wenigstens eine der wenigstens zwei Öffnungen visuell von anderen Teilen der ersten Schicht unterscheidbar ist.

10. Implantat gemäß einem der Ansprüche 4 bis 8, **dadurch gekennzeichnet, dass** wenigstens manche des einen oder der mehreren erhöhten Abschnitte innerhalb der ersten Schicht direkt oder über Bereiche zwischen sich mit der zweiten Schicht miteinander verbunden sind.

11. Chirurgisches Implantat gemäß einem der vorstehenden Ansprüche, **dadurch gekennzeichnet, dass** die erste Schicht wenigstens eines der Materialien ausgewählt aus der folgenden Liste umfasst: Polyalkene, Polypropylen, Polyethylen, fluorierte Polyolefine, Polytetrafluorethylen, PTFE, ePTFE, cPTFE, Polyvinylidenfluorid, Gemische von Polyvinylidenfluorid und Copolymere von Vinylidenfluorid und Hexafluorpropen, Polyamide, Polyurethane, Polyisoprene, Polystyrole, Polysilicone, Polycarbonate, Polyaryletherketone, Polymethacrylsäureester, Polyacrylsäureester, aromatische Polyester, Polyimide, Polyhydroxysäuren, Polylactide, Polyglycolide, Copolymere von Glycolid und Lactid, Copolymere von Glycolid und Lactid in dem Verhältnis von 90:10, Polyhydroxybutyrate, Polyhydroxyvaleriate, Polycaprolactone, Copolymere von Glycolid und ε-Caprolacton, Polydioxanone, Poly-p-dioxanon, synthetische und natürliche Oligo- und Polyaminosäuren, Polyphosphazene, Polyanhydride, Polyorthoester, Polyphosphate, Polyphosphonate, Polyalkohole, Polysaccharide, Polyether, Polyamide, aliphatische Polyester, aromatische Polyester, Polyurethane, Copolymere von polymerisierbaren Stoffen davon, resorbierbare Gläser, Cellulose, bakterielle Cellulose, Allotransplantat, Xenotransplantat, Collagen, Gelatine und Seide.

12. Chirurgisches Implantat gemäß einem der vorstehenden Ansprüche, **dadurch gekennzeichnet, dass** die zweite Schicht ein Material ausgewählt aus der folgenden Liste umfasst: synthetische bioabsorbierbare Polymermaterialien, Polyhydroxysäuren, Polylactide, Polyglycolide, Copolymere von Glycolid und Lactid, Copolymere von Glycolid und Lactid in dem Verhältnis von 90:10, Polyhydroxybutyrate, Polyhydroxyvaleriate, Polycaprolactone, Copolymere von Glycolid und ε-Caprolacton, Polydioxanone, Poly-p-dioxanon, synthetische und natürliche Oligo- und Polyaminosäuren, Polyphosphazene, Polyanhydride, Polyorthoester, Polyphosphate, Polyphosphonate, Polyalkohole, Polysaccharide, Polyether, Collagen, Gelatine, bioabsorbierbare Gelfilme, die mit Omega-3-fettsäuren vernetzt sind, und oxygenierte regenerierte Cellulose, wobei die zweite Schicht gegebenenfalls ferner ein Material ausgewählt aus der folgenden Liste umfasst: synthetische nichtresorbierbare Polymermaterialien, Polyalkene, Polypropylen, Polyethylen, fluorierte Polyolefine, Polytetrafluorethylen, PTFE, ePTFE, cPTFE, Polyvinylidenfluorid, Gemische von Polyvinylidenfluorid und Copolymere von Vinylidenfluorid und Hexafluorpropen, Polyamide, Polyurethane, Polyisoprene, Polystyrole und Polysilicone.

## Revendications

1. Implant chirurgical (1, 11, 51, 61, 71) pour la réparation d'un défaut de la paroi tissulaire ou musculaire comprenant :
- une première couche (2, 12, 52, 62, 72), la première couche comportant une zone centrale et une zone périphérique, et
- une deuxième couche (3, 13, 53, 64),
dans lequel
- la première couche comporte au moins deux ouvertures (55, 63, 74) situées dans la zone périphérique de la première couche,
- les au moins deux ouvertures sont espacées de la périphérie de la première couche,
et **caractérisé en ce que**
- l'implant chirurgical comporte en outre une ou plusieurs sections en relief (5, 15, 54), au moins l'une des une ou plusieurs sections en relief se trouvant dans la première couche et au moins l'une des une ou plusieurs sections en relief se trouvant dans la zone périphérique de la première couche,
- les au moins deux ouvertures situées dans la zone périphérique de la première couche sont dans les sections en relief de la première couche,
- une partie de chacune des au moins deux ouvertures est en relief jusqu'à une hauteur de plus de trois fois l'épaisseur de la première couche par rapport au plan de la première couche, et
- les au moins deux ouvertures sont dimensionnées et formées pour permettre la fixation chirurgicale de l'implant au tissu corporel, les au moins deux ouvertures ayant des dimensions maximales de 2 mm à 2 cm.

2. Implant selon la revendication 1, **caractérisé en ce qu'**il comprend en outre une troisième couche faisant face à la première couche opposée à la deuxième couche étant une couche de film synthétique absorbable comportant des ouvertures, dans lequel la troisième couche a de préférence une épaisseur de 2 µm à 60 µm.

3. Implant selon l'une quelconque des revendications précédentes, **caractérisé en ce que** les au moins deux ouvertures ont des dimensions maximales de 1 mm à 9 mm.

4. Implant selon l'une quelconque des revendications précédentes, **caractérisé en ce qu'**au moins une desdites au moins deux ouvertures situées dans la zone périphérique de la première couche à l'intérieur d'une section en relief de la première couche est une découpe (54) dans une certaine zone ou d'une certaine partie de la section en relief de la première couche.

5. Implant selon l'une quelconque des revendications précédentes, **caractérisé en ce qu'**au moins une des au moins deux ouvertures est prévue sous la forme d'une aile (63) à l'intérieur de la première couche.

6. Implant selon l'une quelconque des revendications précédentes, **caractérisé en ce que** la deuxième couche présente une ou plusieurs sections en relief.

7. Implant selon l'une quelconque des revendications précédentes, **caractérisé en ce que** la première et/ou la deuxième couche sont au moins partiellement constituées d'une maille chirurgicale, d'une bande, d'un cordon, d'un ruban de film, d'une structure tissée, d'un matériau tricoté, d'un tissu et/ou d'un molleton, la deuxième couche comprenant de préférence une maille, un molleton, un film, une structure tissée, une structure non tissée, un film ou un film perforé.

8. Implant selon l'une quelconque des revendications précédentes, **caractérisé en ce qu'**il existe une ou plusieurs sections en relief dans la zone centrale de la première couche, de préférence dans lequel lesdites sections en relief ont une zone de base qui est de forme ronde, triangulaire, tétragonale, pentagonale, hexagonale, symétrique, asymétrique, circulaire, elliptique, ovale, carrée, rectangulaire, trapézoïdale ou rhomboïdale, et la zone de tête est de forme plate, pointue, ronde, concave ou convexe.

9. Implant selon l'une quelconque des revendications précédentes, **caractérisé en ce qu'**au moins une des au moins deux ouvertures est visuellement différenciée des autres parties de la première couche.

10. Implant selon l'une quelconque des revendications 4 à 8, **caractérisé en ce qu'**au moins certaines de la ou des sections en relief au sein de la première couche sont reliées entre elles directement ou par le biais de zones entre elles avec la deuxième couche.

11. Implant chirurgical selon l'une quelconque des revendications précédentes, **caractérisé en ce que** la première couche comprend au moins un des matériaux choisis dans la liste suivante : polyalcènes, polypropylène, polyéthylène, polyoléfines fluorées, polytétrafluoroéthylène, PTFE, ePTFE, cPTFE, polyfluorure de vinylidène, mélanges de polyfluorure de vinylidène et de copolymères de fluorure de vinylidène et d'hexafluoropropène, polyamides, polyuréthanes, polyisoprènes, polystyrènes, polysilicones, polycarbonates, polyaryléthercétones, esters d'acide polyméthacrylique, esters d'acide polyacrylique, polyesters aromatiques, polyimides, acides polyhydroxyliques, polylactides, polyglycolides, copolymères de glycolide et de lactide, copolymères de glycolide et de lactide dans le rapport 90:10, polyhydroxybutyrates, polyhydroxyvalériates, polycaprolactones, copolymères de glycolide et de ε-caprolactone, polydioxanones, poly-p-dioxanone, acides oligoaminés et polyaminés synthétiques et naturels, polyphosphazènes, polyanhydrides, polyorthoesters, polyphosphates, polyphosphonates, polyalcools, polysaccharides, polyéthers, polyamides, polyesters aliphatiques, polyesters aromatiques, polyuréthanes, copolymères de substances polymérisables associées, verres résorbables, cellulose, cellulose bactérienne, allogreffe, xénogreffe, collagène, gélatine et soie.

12. Implant chirurgical selon l'une quelconque des revendications précédentes, **caractérisé en ce que** la deuxième couche comprend un matériau choisi dans la liste suivante : matériaux polymères bioabsorbables synthétiques, acides polyhydroxyliques, polylactides, polyglycolides, copolymères de glycolide et de lactide, copolymères de glycolide et de lactide dans le rapport 90:10, polyhydroxybutyrates, polyhydroxyvalériates, polycaprolactones, copolymères de glycolide et de ε-caprolactone, polydioxanones, poly-p-dioxanone, acides oligoaminés et polyaminés synthétiques et naturels, polyphosphazènes, polyanhydrides, polyorthoesters, polyphosphates, polyphosphonates, polyalcools, polysaccharides, polyéthers, collagène, gélatine, films de gel bioabsorbables réticulés avec des acides gras oméga 3, et cellulose régénérée oxygénée, dans lequel ladite deuxième couche comprend en outre éventuellement un matériau choisi dans la liste suivante : matériaux polymères synthétiques non résorbables, polyalcènes, polypropylène, polyéthylène, polyoléfines fluorées, polytétrafluoroéthylène, PTFE, ePTFE, cPTFE, fpolyluorure de vinylidène, mélanges de polyfluorure de vinylidène et de copolymères de fluorure de vinylidène et d'hexafluoropropène, polyamides, polyuréthanes, polyisoprènes, polystyrènes et polysilicones.
